(19) **Europäisches Patentamt European Patent Office Office européen des brevets**

(11) **EP 2 795 390 B1**

(12) **FASCICULE DE BREVET EUROPEEN**

(45) Date de publication et mention
de la délivrance du brevet:
**28.03.2018 Bulletin 2018/13**

(51) Int Cl.:
*G01N 33/483* (2006.01)    *G02B 21/06* (2006.01)
*G01N 21/64* (2006.01)    *G01N 21/65* (2006.01)
*G02B 21/26* (2006.01)    *G02B 21/00* (2006.01)
*G02B 21/36* (2006.01)

(21) Numéro de dépôt: **12816697.2**

(22) Date de dépôt: **03.12.2012**

(86) Numéro de dépôt international:
**PCT/FR2012/052784**

(87) Numéro de publication internationale:
**WO 2013/093275 (27.06.2013 Gazette 2013/26)**

(54) **MICROSCOPIE OPTIQUE NON-LINÉAIRE QUANTITATIVE UTILISANT UN FAISCEAU MIS EN FORME**

QUANTITATIVE NICHTLINEARE OPTISCHE MIKROSKOPIE MIT EINEM GEFORMTEN STRAHL

QUANTITATIVE NONLINEAR OPTICAL MICROSCOPY USING A SHAPED BEAM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.12.2011 FR 1162056**

(43) Date de publication de la demande:
**29.10.2014 Bulletin 2014/44**

(73) Titulaires:
• **Ecole Polytechnique**
**91120 Palaiseau (FR)**
• **Centre National de la Recherche Scientifique
(C.N.R.S.)
75016 Paris (FR)**

(72) Inventeurs:
• **SCHANNE-KLEIN, Marie-Claire**
**91370 Verrières Le Buisson (FR)**
• **BEAUREPAIRE, Emmanuel, Jean-Marc**
**91120 Palaiseau (FR)**
• **STRUPLER, Mathias**
**Montreal, QC H2G 1W5 (CA)**
• **DEBARRE, Delphine**
**75014 Paris (FR)**
• **OLIVIER, Nicolas**
**90000 Belfort (FR)**
• **MAHOU, Pierre**
**91300 Massy (FR)**

(74) Mandataire: **Nony**
**11 rue Saint-Georges
75009 Paris (FR)**

(56) Documents cités:
EP-A2- 0 627 643    EP-A2- 2 282 230
WO-A1-01/35325    DE-A1-102008 049 886
JP-A- 2006 113 484    US-A1- 2003 231 791
US-A1- 2005 258 375    US-A1- 2010 150 472
US-A1- 2010 253 774    US-B1- 7 194 118

• BOTCHERBY E J ET AL: "Scanning two photon
fluorescence microscopy with extended depth of
field", OPTICS COMMUNICATIONS,
NORTH-HOLLAND PUBLISHING CO.
AMSTERDAM, NL, vol. 268, no. 2, 15 décembre
2006 (2006-12-15), pages 253-260, XP028081032,
ISSN: 0030-4018, DOI:
10.1016/J.OPTCOM.2006.07.026 [extrait le
2006-12-15] cité dans la demande

**Description**

La présente invention se rapporte à un microscope optique non-linéaire ou multiphoton équipé pour réaliser une imagerie de bonne résolution latérale. Elle trouve une application particulièrement intéressante, mais non limitative, dans l'imagerie quantitative du collagène ou du contenu lipidique.

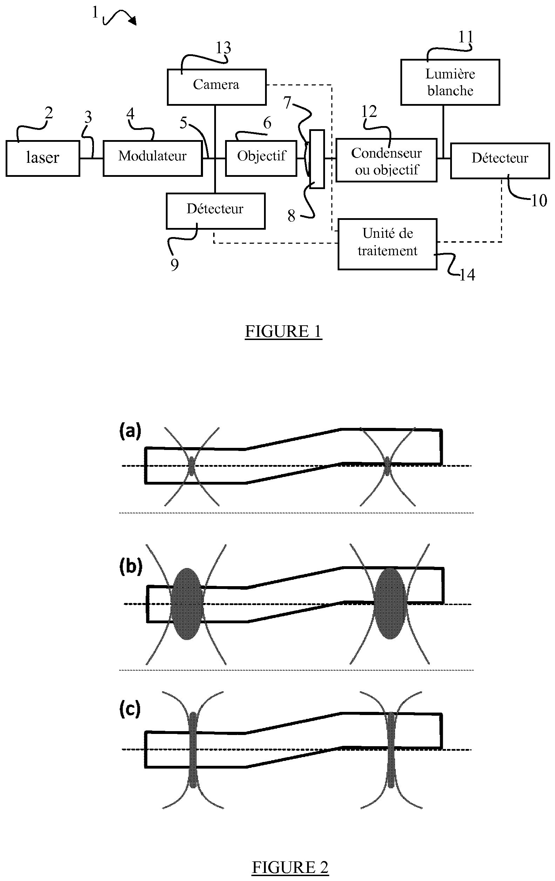

D'une façon générale, la résolution des images en microscopie optique non-linéaire est liée au carré ou au cube de la distribution d'intensité excitatrice. Les microscopes optiques non-linéaires usuels utilisent un profil d'excitation de type Gaussien qui est la distribution naturelle obtenue avec un faisceau laser non mis en forme (profil d'intensité gaussien et phase plate à l'entrée de l'objectif de focalisation). La résolution est alors de 300-400 nm dans la direction latérale et de 1-3 microns dans la direction axiale, dans des conditions d'excitation et de détection usuelles (ouverture numérique proche de 1, longueur d'onde d'excitation proche de 1 $\mu$m). Cette bonne résolution axiale est équivalente à une faible profondeur de champ et ne permet pas d'imager en une seule fois une coupe mince de tissu dont l'épaisseur est de quelques microns (2-7 $\mu$m typiquement). Ceci est d'autant plus critique que le champ imagé est plus grand. En effet, il est souvent essentiel, notamment en histologie, d'imager de grandes zones d'intérêt (typiquement centimétrique) en déplaçant l'échantillon et en reconstituant une mosaïque d'images. La zone imagée doit alors rester plane et horizontale à l'échelle de cette résolution axiale de quelques microns pour que la coupe soit imagée correctement. Ceci n'est en pratique jamais le cas car les lames de microscope ne sont pas planes à cette échelle, les coupes ne sont jamais parfaitement étalées, et il est très difficile d'aligner la lame parallèlement au plan focal à quelques microns près sur des distances de l'ordre du centimètre. Sur les figures 2a et 2b, on voit une imagerie multiphoton d'une coupe mince de tissu (typiquement 5 $\mu$m d'épaisseur) présentant des défauts de planéité ou d'horizontalité. La figure 2a est conforme à l'art antérieur et concerne l'utilisation d'un faisceau gaussien fortement focalisé (ouverture numérique > 0,5) : la résolution latérale est satisfaisante, mais la profondeur de champ est inférieure à l'épaisseur de la coupe, et par conséquent le volume imagé sort parfois du tissu. La figure 2b est conforme à l'art antérieur et concerne l'utilisation d'un faisceau gaussien peu focalisé (ouverture numérique <0,5) : la profondeur de champ est plus importante, ce qui permet de maintenir le volume imagé dans le tissu, mais la résolution latérale est fortement dégradée.

On connaît par ailleurs dans la littérature des publications enseignant l'utilisation de faisceaux de Bessel pour étendre la profondeur de champ tout en maintenant la bonne résolution latérale d'un faisceau d'excitation. Parmi ces publications, on distingue :

- "Two-photon excitation fluorescence microscopy with a high depth of field using an axicon"; Pascal Dufour et al., Applied Optics/vol. 45, No. 36/ 20 décembre 2006;
- "Scanning two photon fluorescence microscopy with extended depth of field"; E. J. Botcherby et al., Optics Communications 268 (2006) 253-260;
- "Rapid three-dimensional isotropic imaging of living cells using Bessel beam plane illumination"; Thomas A Planchon et al., Nature Methods/vol.8 No.5/May 2011; et
- "Two-photon microscopy with simultaneous standard and extended depth of field using a tunable acoustic gradient-index lens"; Nicolas Olivier et al., Optics Letters/vol.34, No.11/june 1, 2009.

La présente invention a pour but la réalisation d'imagerie de bonne résolution latérale notamment sur des lames ne présentant pas une parfaite planéité et horizontalité.

Un autre but de l'invention est de proposer un système d'imagerie de grande sensibilité et spécificité. La présente invention a encore pour but un système d'imagerie capable d'acquérir rapidement des grandes zones d'intérêt. On atteint au moins l'un des objectifs précités avec un dispositif optique selon la revendication 1. Le dispositif ainsi défini peut être considéré comme une combinaison intelligente d'un scanner de lames avec un microscope non linéaire, cette combinaison étant obtenue par une disposition particulière de différents composants les uns par rapport aux autres.

Les faisceaux produits peuvent être des faisceaux intermédiaires entre les faisceaux gaussiens et les faisceaux de Bessel. De tels faisceaux peuvent notamment être générés en se basant sur l'enseignement de l'ouvrage « Principles of nano-optics », L. Novotny and B. Hecht, Cambridge Univ Press, 2006, en particulier les chapitres 1 à 5 pour la mise en forme spatiale de faisceaux.

Mais, de préférence, le faisceau modulé est un faisceau de Bessel produit sous la forme d'une distribution annulaire d'intensité sur la pupille arrière de l'objectif.

Selon l'invention, l'ensemble scanner de lames peut comprendre :

- une source de lumière blanche,
- un condenseur pour guider la lumière blanche vers la lame, et
- une caméra pour produire une image à partir de la lumière provenant de la lame.

On prévoit de préférence une unité de traitement intégrée ou distante prévue pour récupérer les signaux provenant du détecteur et reconstruire les images multiphoton, de façon à les associer à des images conventionnelles provenant de la caméra. Les deux images sont recalées et combinées de manière automatique, après une étape d'étalonnage sur des échantillons modèles.

Avec le microscope selon l'invention, il est désormais possible, notamment en histologie, d'imager de grandes zones d'intérêt, typiquement centimétrique, en déplaçant la lame supportant l'échantillon et en reconstituant une mosaïque d'images. Ce déplacement est notamment obtenu par le balayage de la platine et donc de la lame. La lame est placée sur la platine motorisée avec chargement automatisé de lames. Chaque lame porte un échantillon à analyser. La platine est motorisée en deux dimensions de sorte que la totalité de la surface de la lame peut être balayée. La platine peut aussi être motorisée dans la direction axiale en complément ou en remplacement du déplacement motorisé axial de l'objectif. De préférence, pour limiter encore la durée d'acquisition tout en conservant une bonne résolution, on peut acquérir plusieurs images numériques de différentes parties disjointes de la lame. Ces images permettant ensuite de reconstituer une seule image de l'ensemble de la lame ou de différentes régions d'intérêt de l'échantillon.

Avantageusement, l'invention concerne l'implémentation d'une imagerie optique non-linéaire ou multiphoton reposant sur l'utilisation de faisceaux mis en forme, en particulier les faisceaux dits de Bessel, pour l'excitation des processus optiques non-linéaires en microscopie multiphoton. Le volume d'excitation obtenu par un faisceau de Bessel est étendu dans la direction axiale par rapport à celui obtenu avec un faisceau Gaussien classique, mais présente une résolution latérale similaire. Le volume d'excitation prend ainsi la forme d'un tube dont la longueur est ajustable de quelques microns à plusieurs centaines de microns. Le microscope selon l'invention reposant sur ce type d'excitation permet donc d'imager en une seule fois des systèmes épais jusqu'à plusieurs centaines de micromètres avec une résolution latérale sub-micrométrique, et ceci de manière robuste dans des milieux diffusants.

Plus généralement, la mise en forme du faisceau peut être adaptée aux spécificités des échantillons étudiés en ajustant la distribution d'intensité et de phase sur la pupille arrière de l'objectif. On produit ainsi un faisceau étendu axialement et confiné dans une ou deux directions latérales après focalisation par l'objectif.

Ce type de mise en forme peut être complété par l'utilisation d'un système multipoints. Plusieurs faisceaux étendus axialement et confinés latéralement sont alors envoyés simultanément sur la lame, et le détecteur ponctuel est remplacé par une caméra.

Selon une caractéristique avantageuse de l'invention, les moyens de détection comprennent un condenseur et un détecteur disposé en aval de la lame par rapport à la propagation du faisceau laser pulsé de façon à détecter des signaux de transmission. Le condenseur peut être le condenseur utilisé pour guider la source de lumière incohérente vers la lame. Il peut être remplacé par un objectif de forte ouverture numérique.

On peut également prévoir que les moyens de détection comprennent un détecteur, notamment en plus du détecteur pour la transmission, disposé de façon à détecter des signaux émis vers l'arrière ou réfléchis depuis la lame au moyen de l'objectif utilisé pour l'excitation. Pour ce faire, on peut utiliser un filtre dichroïque dans le chemin optique du faisceau laser en amont de la lame de façon à diriger les signaux réfléchis vers ce détecteur.

De préférence, l'objectif et le condenseur sont respectivement disposés de part et d'autre de la lame. Par exemple, la lumière du scanner de lames peut éclairer le dessous de la lame alors que le faisceau laser atteint le dessus de la lame où se trouve l'échantillon. Si le faisceau laser pulsé pour l'excitation multiphoton et la source de lumière pour le scanner de lame sont disposés du même côté de l'échantillon, l'objectif utilisé pour guider le faisceau de Bessel est utilisé pour guider la lumière vers la lame à la place du condenseur. Un deuxième objectif est alors placé de l'autre côté de l'échantillon pour collecter la lumière transmise et la guider vers la caméra. L'ensemble "scanner de lames" peut aussi être réduit au support de lame avec déplacement motorisé et chargement automatique, sans visualisation en lumière transmise. En d'autres termes, on peut soit supprimer la visualisation en lumière blanche, c'est-à-dire ne conserver du scanner de lame que la platine motorisée et le chargement automatisé, soit mettre les systèmes multiphoton et lumière blanche du même côté en utilisant deux objectifs. Ces deux dernières solutions sont moins pratiques.

Selon une caractéristique avantageuse de l'invention, l'objectif présente une ouverture numérique supérieure à 0,5 de façon à fortement focaliser le faisceau de Bessel et obtenir une résolution latérale très satisfaisante.

De préférence, le faisceau laser pulsé est un train d'impulsions femtoseconde, par exemple autour de 100 fs avec un taux de répétition supérieur à une dizaine de MHz, par exemple autour de 100MHz, et une puissance de plusieurs centaines de milliwatts. D'une façon générale, on utilise un faisceau laser capable de générer les phénomènes non linéaires attendus en fonction de l'échantillon à analyser. En particulier, on utilise des milieux dans lesquels on met en oeuvre la génération de second harmonique (SHG) ou de troisième harmonique (THG), la fluorescence excitée à deux photons (2PEF), la diffusion Raman anti-Stokes cohérente (CARS), la diffusion Raman stimulée (SRS), les mélanges à quatre ondes (FWM), ou d'autres contrastes optiques non-linéaires cohérents ou incohérents.

Selon une caractéristique avantageuse de l'invention, le modulateur spatial de lumière comprend un élément permettant la mise en forme du faisceau de Bessel. Cet élément peut notamment être choisi parmi :

- un axicon ou lentille conique ;
- un élément diffractif ;

- un modulateur à cristaux liquides ; et
- une lentille liquide modulable de type acoustique.

De préférence, on prévoit en outre des moyens de balayage du faisceau laser. Ce balayage se fait sur un plan et peut être complété par un déplacement axial de l'objectif de façon à réaliser des images en 3D. Ces moyens de balayage peuvent comprendre des éléments galvanométriques, piézoélectriques, ou autres, déplaçant la source laser, et/ou intégrés dans l'un des éléments traversés par le faisceau laser et/ou intégrés dans un élément optique supplémentaire placé dans le chemin optique du faisceau laser. La combinaison du balayage du faisceau laser avec le balayage de la platine motorisée permet d'obtenir des images précises, d'une grande résolution et de différentes zones d'intérêt.

Selon un autre aspect de l'invention, il est proposé au moins l'une des applications suivantes mettant en oeuvre le microscope selon l'invention :

- application dans l'imagerie quantitative du collagène en utilisant le signal de génération de second harmonique (SHG) intrinsèque du collagène fibrillaire ;
- application dans l'imagerie quantitative du contenu lipidique d'un tissu en utilisant un signal de type génération de troisième harmonique (THG), diffusion Raman anti-Stokes cohérente (CARS), diffusion Raman stimulée (SRS) et/ou les mélanges à quatre ondes (FWM), et
- application dans l'imagerie quantitative cellulaire ou extra cellulaire, comme par exemple l'élastine, en utilisant un signal de fluorescence excitée à deux ou trois photons.

En d'autres termes, un tel dispositif peut en particulier s'appliquer à l'imagerie quantitative du collagène dans des biopsies non colorées en utilisant le signal SHG intrinsèque du collagène fibrillaire. En effet, il permet de mesurer quantitativement l'accumulation de collagène fibrillaire sur de grandes zones d'intérêt d'un tissu en s'affranchissant à la fois des problèmes de planéité et d'horizontalité de la coupe histologique.

Il a en effet été démontré que la microscopie SHG est une sonde structurelle de l'organisation fibrillaire du collagène. Or il est indispensable de quantifier l'accumulation de collagène fibrillaire dans de nombreuses pathologies liées à des remodelages tissulaires. C'est le cas par exemple des pathologies fibrosantes (fibroses du foie, du rein, du poumon) dont les maladies chroniques des implants. Il a ainsi été montré que l'accumulation du collagène fibrillaire permet de diagnostiquer une évolution défavorable de ces pathologies. Plus généralement, il est intéressant de quantifier la présence de collagène fibrillaire dans l'ensemble des remodelages tissulaires, qu'ils soient d'origine immunologique, cancéreux, infectieux, etc...

L'intérêt de la microscopie SHG dans ce contexte réside dans le fait qu'elle est très spécifique des collagènes fibrillaires tout en ne nécessitant pas de coloration ou de marquage, ce qui assure une excellente reproductibilité. Ceci est un avantage déterminant pour réaliser des études multi-centres pour l'évaluation de nouveaux traitements ou pour la recherche fondamentale. Mais l'accumulation de collagène fibrillaire est en général fortement hétérogène dans les organes atteints et demande l'imagerie de grandes zones d'intérêt (au moins centimétriques). Le dispositif de l'invention permet de réaliser cela tout en préservant une résolution latérale sub-micrométrique qui maintient une bonne sensibilité pour la détection de petites fibrilles de collagène. On mesure ainsi quantitativement l'extension du réseau de collagène fibrillaire sur une grande zone d'intérêt en acquérant une seule image dans la direction axiale, c'est-à-dire de manière bien plus rapide et directe qu'avec des faisceaux gaussiens pour lesquels il est nécessaire d'acquérir une pile de plusieurs images dans la direction axiale. L'invention pourrait de plus permettre d'exploiter l'intensité du signal SHG pour mesurer quantitativement le nombre de fibrilles de collagène alignées parallèlement dans le volume focal. Ceci nécessiterait de mener des modélisations de la construction de signaux cohérents avec des faisceaux mis en forme en tenant compte de la variation de la phase du signal excitateur le long du volume focal.

Un tel microscope pour signal SHG permet ainsi un diagnostic quantitatif de pathologies fibrosantes ou de toute pathologie impliquant l'accumulation de collagène fibrillaire.

De même, le dispositif de l'invention peut servir à caractériser rapidement le nombre et la taille des gouttelettes lipidiques présentes dans une coupe de tissu ou même un petit organisme (type *c elegans*) en microscopie par génération de troisième harmonique (THG) ou microscopie Raman cohérente (CARS, SRS, etc.). Ces techniques ne nécessitent pas de coloration ou de marquage, ce qui améliore fortement la reproductibilité des mesures.

D'autres avantages et caractéristiques de l'invention apparaîtront à l'examen de la description détaillée d'un mode de mise en oeuvre nullement limitatif, et des dessins annexés, sur lesquels :

- La figure 1 est une vue schématique d'un dispositif selon l'invention ;
- Les figures 2a à 2c sont des vues schématiques d'imageries multiphoton d'une coupe mince de tissu, typiquement 5 μm d'épaisseur, présentant des défauts de planéité ou d'horizontalité ; la figure 2a concerne l'utilisation d'un faisceau gaussien fortement focalisé selon l'art antérieur, on distingue que la profondeur de champ est plus faible que l'épaisseur du tissu ; le volume focal se situe entièrement à l'intérieur du tissu ou peut en sortir à cause des défauts de planéité ou d'horizontalité ;

- La figure 2b concerne l'utilisation d'un faisceau gaussien peu focalisé selon l'art antérieur, on distingue un volume focal important, correspondant à une faible résolution latérale, et couvrant toute l'épaisseur du tissu ;
- La figure 2c concerne l'utilisation d'un faisceau de Bessel fortement focalisé selon la présente invention, on distingue un volume focal fin, en forme de tube, correspondant à une grande résolution latérale, et couvrant toute l'épaisseur du tissu même en cas de défaut de planéité et d'horizontalité ;
- Les figures 3a et 3b sont des vues schématiques d'imageries multiphoton d'une coupe mince de tissu ou d'une cellule, typiquement de quelques μm d'épaisseur ; la figure 3a concerne l'utilisation d'un faisceau gaussien fortement focalisé selon l'art antérieur ; la figure 3b concerne l'utilisation d'un faisceau de Bessel fortement focalisé selon la présente invention ;
- La figure 4 illustre plusieurs images comparatives entre une image obtenue à partir d'une seule acquisition avec un faisceau de Bessel dans le dispositif selon l'invention et une image obtenue à partir de plusieurs acquisitions à différentes profondeurs avec un faisceau gaussien.

En référence à la figure 1, on voit un dispositif 1 selon l'invention comprenant une source laser 2 émettant un faisceau d'excitation 3, telle que par exemple un laser pulsé femtoseconde. Cette source laser 2 peut être ajustable, notamment en intensité ou temporellement (largeur spectrale, durée, cadence et phase spectrale des impulsions, ...). A titre d'exemple, il s'agit d'un faisceau laser pulsé délivrant des impulsions d'environ 100 fs, avec un taux de répétition de 100MHz et une puissance moyenne de quelques centaines de milliwatts.

Le faisceau d'excitation 3 est mis en forme spatialement par un modulateur spatial de lumière 4. Ce modulateur peut comprendre un axicon ou une lentille conique, un élément diffractif, un modulateur à cristaux liquides, une lentille liquide acoustique ou tout autre modulateur spatial de lumière capable d'opérer une mise en forme de type Bessel.

La présente invention se réfère explicitement aux publications suivantes qui fournissent des informations plus détaillées relatives à l'élaboration d'un faisceau de Bessel :

- "Two-photon excitation fluorescence microscopy with a high depth of field using an axicon"; Pascal Dufour et al., Applied Optics/vol. 45, No. 36/ 20 décembre 2006; cette publication enseigne l'utilisation d'un axicon;
- "Scanning two photon fluorescence microscopy with extended depth of field"; E. J. Botcherby et al., Optics Communications 268 (2006) 253-260; on utilise un élément diffractif pour avoir une illumination annulaire et générer un faisceau de Bessel ;
- "Rapid three-dimensional isotropic imaging of living cells using Bessel beam plane illumination"; Thomas A Planchon et al., Nature Methods/vol.8 No.5/May 2011; on utilise ici une géométrie orthogonale ; et
- "Two-photon microscopy with simultaneous standard and extended depth of field using a tunable acoustic gradient-index lens"; Nicolas Olivier et al., Optics Letters/vol.34, No.11/june 1, 2009; on utilise dans cette publication une lentille acousto-optique.

Les éléments divulgués dans ces publications sont incorporés dans la présente invention dans la mesure où ces éléments ne sont pas incompatibles avec l'enseignement de la présente invention.

Le faisceau de Bessel 5 présente à l'entrée de l'objectif 6 une distribution d'intensité moyenne sous la forme d'un anneau. On forme en fait un anneau de lumière sur la pupille arrière de l'objectif 6. Le faisceau de Bessel est ensuite focalisé sur un échantillon 7 posé sur la lame 8. Ce faisceau de Bessel est à profondeur de champ étendu. Les signaux non-linéaires provenant de l'échantillon 7 sont captés en réflexion, ou épidétection, par le détecteur 9 et/ou en transmission par le détecteur 10. La présente invention est notamment remarquable par le fait que les éléments de microscopie optique non-linéaire qui viennent d'être décrits sont combinés avec un scanner de lames d'histologie comprenant une source de lumière blanche 11 éclairant un objectif ou un condenseur 12. Cette lumière blanche est ensuite guidée vers l'échantillon 7 à travers la lame 8. La caméra numérique 13 permet de capter des images numériques de l'échantillon à partir de la lumière blanche provenant de l'échantillon.

On prévoit une unité de traitement 14 équipée de moyens logiciels et matériels nécessaires et connus de l'homme du métier pour piloter différents composants du dispositif 1 selon l'invention, traiter les images numériques de la caméra numérique 13 et les signaux provenant des détecteurs 9 et/ou 10, puis afficher des images sur un écran.

La lame 8 est portée par une platine motorisée avec chargement automatisé. L'acquisition d'une image est obtenue par balayage de la platine motorisée.

Le dispositif selon l'invention peut comprendre des moyens de balayage (non représenté), qui permettent de balayer latéralement l'échantillon avec le faisceau de Bessel 5 sortant du modulateur spatial de lumière 4. Ces moyens de balayage peuvent comprendre par exemple des scanners à miroir non résonnants et/ou des éléments acousto-optiques entre le modulateur et l'objectif. Ils sont avantageusement commandés en combinaison avec la commande de balayage de la platine motorisée.

Sur la figure 2c, on voit une imagerie multiphoton d'une coupe mince de tissu, typiquement de 5 μm d'épaisseur,

présentant des défauts de planéité ou d'horizontalité. On constate qu'un faisceau de Bessel fortement focalisé, avec une ouverture numérique supérieure à 0,5, permet une résolution latérale satisfaisante, et une profondeur de champ supérieure à l'épaisseur de la coupe, ce qui permet de pallier tout défaut de planéité.

Un des avantages du dispositif selon la présente invention est la rapidité à laquelle on peut obtenir des images. En effet, l'utilisation de ces faisceaux de Bessel en microscopie multiphoton permet d'obtenir une image en une seule acquisition, pour les raisons suivantes :

(i) le volume d'excitation étendu dans la direction axiale permet de sonder l'intégralité d'une coupe de quelques micromètres d'épaisseur, même sur de grandes régions d'intérêt présentant des défauts de planéité ou d'horizontalité (voir figure 2c) ;
(ii) la résolution latérale est préservée à un niveau similaire à celle obtenue avec des faisceaux gaussiens, ce qui permet une bonne sensibilité à une échelle submicrométrique ; Ceci n'est pas possible si on utilise des faisceaux gaussiens moins focalisés comme sur la figure 2b de l'art antérieur ;
(iii) le niveau de signal obtenu en chaque plan n'est pas significativement diminué par l'extension axiale du volume d'excitation car l'intensité d'excitation dans chaque plan n'est pas diminuée.

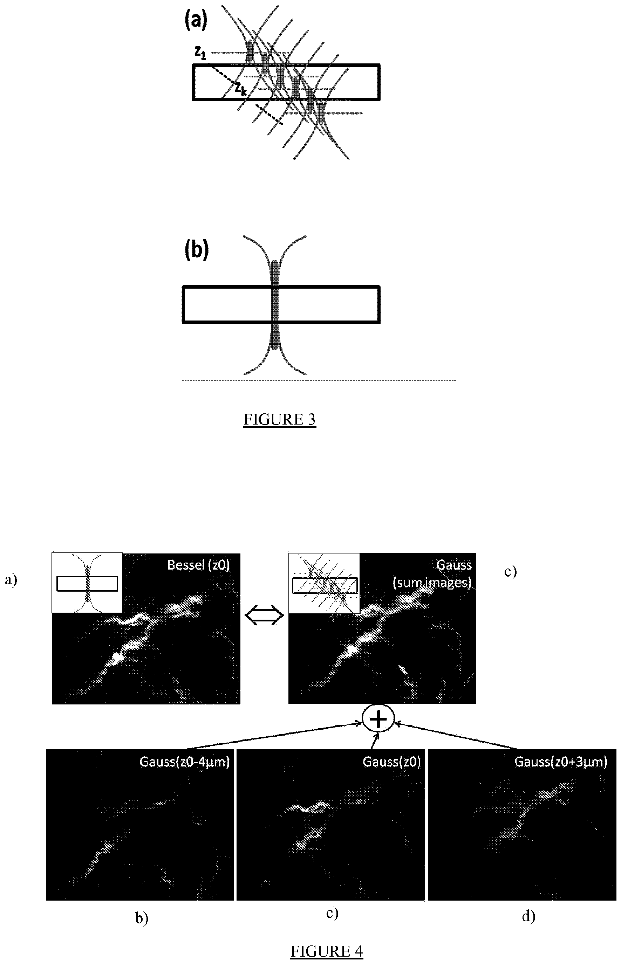

Sur les figures 3a et 3b sont représentées des vues schématiques des fonctions d'étalement du point (PSF, « point spread function ») pour de l'imagerie multiphoton d'une coupe mince de tissu ou d'une cellule, typiquement de quelques $\mu$m d'épaisseur, avec une bonne résolution latérale (ouverture numérique > 0,5). La fonction d'étalement du point pour un faisceau gaussien selon l'art antérieur est illustrée sur la figure 3a.

Il est nécessaire de sommer les contributions de plusieurs images décalées axialement selon l'équation suivante :

$$I_{NL} = \sum_{k=1}^{p} I_{NL}(z_k) = \sum_{k=1}^{p} \left| \int_{z_k} \chi^{(n)}(z) E_\omega^n(z) e^{ni\varphi(z)} \, dz \right|^2$$

Où $I_{NL}$ est l'intensité du signal optique non-linéaire sur toute l'épaisseur de l'échantillon, $I_{NL}(z_k)$ est l'intensité du signal optique non-linéaire détecté lorsque le faisceau d'excitation est focalisé à la profondeur $z_k$ où k varie de 1 à p, $\chi^{(n)}(z)$ est la susceptibilité optique non-linéaire effective d'ordre n à la profondeur z, $E_\omega^n(z)$ est l'amplitude du champ électrique excitateur de fréquence $\omega$ à la profondeur z, élevé à la puissance n, $\varphi(z)$ est la phase du champ électrique

excitateur de fréquence $\omega$ à la profondeur z et $\int_{z_k}$ correspond à l'intégrale sur le volume d'excitation focalisé à la

profondeur

$z_k$. n est un entier par exemple entre 2 et 4, par exemple 2 pour le SHG, 3 pour le THG, CARS, SRS, 2PEF, et 4 pour 3PEF.

Alors qu'avec l'utilisation d'un faisceau de Bessel, une seule image est nécessaire car le faisceau est tellement étendu axialement qu'il va au-delà de l'épaisseur du tissu tout en conservant une excellente résolution latérale comme on le voit sur la figure 3b. Une seule contribution selon l'équation suivante est nécessaire :

$$I_{NL} = \left| \int \chi^{(n)}(z) E_\omega^n(z) e^{ni\varphi(z)} dz \right|^2$$

Où l'intégrale est effectuée sur l'épaisseur totale de l'échantillon.

En référence à la figure 4, on voit des images en microscopie SHG d'une lame histologique de 15$\mu$m d'épaisseur de rein de souris fibrosé. L'excitation est faite soit avec un faisceau de Bessel produit avec un axicon selon le principe illustré sur la figure 3b, voir l'image 4a ; soit avec un faisceau Gaussien selon le principe illustré sur la figure 3a, voir les images 4b à 4d assemblées pour obtenir une image finale 4e. L'image 4a obtenue avec le faisceau de Bessel révèle les fibres de collagène présentes sur l'ensemble de l'épaisseur de la lame, contrairement aux images 4b à 4e obtenues avec un faisceau Gaussien.

D'une façon générale, l'invention peut servir à mesurer rapidement toute source de contraste en microscopie multiphoton sur des échantillons minces ou semi-épais (typiquement du micron à plusieurs centaines de micromètres). Les signaux cohérents utilisés (type SHG, THG, CARS, SRS, FWM), émis principalement vers l'avant, sont détectés directement sur une voie en transmission, ou bien ils sont détectés sur une voie en réflexion, soit directement et grâce aux rétrodiffusions et rétroréflexions de l'échantillon, soit en utilisant un miroir de renvoi. Les signaux non cohérents (2PEF, 3PEF) peuvent être détectés indifféremment dans une voie en réflexion ou en transmission. L'acquisition de signaux cohérents peut être combinée à l'acquisition des signaux 2PEF/3PEF pour compter les cellules, par exemple en utilisant un marqueur fluorescent couramment utilisé tel que le DAPI (Di-Amidino-Phenyl-Indol), pour imager la morphologie du tissu ou pour visualiser d'autres molécules ou structures d'intérêt. Cette fluorescence peut résulter de marquages immunochimiques ou histologiques, de modifications génétiques (fusions de protéines fluorescentes) ou de chromophores endogènes. D'une manière générale, plusieurs signaux cohérents et incohérents peuvent être détectés simultanément pour localiser et quantifier simultanément diverses composantes d'intérêt du tissu, par exemple (i) la localisation et la quantification du collagène fibrillaire par SHG et du contenu lipidique par microscopie THG, CARS, SRS ou FWM, ou (ii) la localisation et la quantification du collagène fibrillaire par SHG et de certains types cellulaires par le signal 2PEF après un immunomarquage adéquat.

En d'autres termes, l'invention tire profit d'un scanner de lames multiphoton, c'est-à-dire d'un dispositif automatisé pour l'imagerie de lames histologiques sur de grandes zones d'intérêt. Dans ce dispositif, le balayage du faisceau laser peut être combiné au balayage du support des lames ou bien intégralement remplacé par celui-ci. Dans ce cas, la platine supportant les lames est balayée finement (avec un pas sub-micrométrique) sur une amplitude de quelques centimètres. Dans cette implémentation, l'invention s'applique à des lames paraffinées ou à des coupes cryogéniques, fixées ou pas, entre lame et lamelle. Le passage d'un type d'échantillon à un autre (présence de paraffine ou pas, présence de lamelle couvre-objet ou pas, épaisseur de coupe différentes...) correspond à des réglages différents de la mise en forme des faisceaux utilisés, qui peuvent être automatisés. Le chargement et l'alignement grossier des lames parallèlement au plan focal peuvent aussi être automatisés.

Par rapport aux dispositifs multiphoton conventionnels, le dispositif selon la présente invention comporte au moins les avantages suivants : rapidité des mesures quantitatives, élimination des problèmes liés aux défauts de planéité et d'horizontalité des coupes minces.

Par rapport aux scanners de lames conventionnels, le dispositif selon la présente invention comporte au moins les avantages suivants : spécificité et fort contraste des diffuseurs non-linéaires par rapport aux colorations histologiques, meilleure reproductibilité grâce à l'absence de marquage.

Bien sûr, l'invention n'est pas limitée aux exemples qui viennent d'être décrits et de nombreux aménagements peuvent être apportés à ces exemples sans sortir du cadre de l'invention.

**Revendications**

1.   Dispositif optique comprenant :

- un ensemble scanner de lames histologiques comprenant une platine motorisée, destinée à supporter une lame (8) sur laquelle est placé un échantillon (7), et un microscope optique non-linéaire comprenant :

- une source (2) de faisceau laser pulsé (3),
- un modulateur spatial (4) de lumière pour moduler le profil spatial du faisceau laser pulsé,
- un objectif (6) pour guider le faisceau modulé (5) vers la lame (8), et
- des moyens de détection (9, 10) pour recueillir des signaux cohérents et incohérents provenant de l'échantillon (7),

dans lequel le modulateur spatial (4) de lumière est conçu pour moduler l'intensité et/ou la phase du faisceau laser pulsé (3) sur la pupille arrière de l'objectif (6) de façon à produire un faisceau étendu axialement et confiné dans une ou deux directions latérales après focalisation par l'objectif (6).

2.   Dispositif selon la revendication 1, **caractérisé en ce que** le faisceau modulé est un faisceau de Bessel (5) produit sous la forme d'une distribution annulaire d'intensité sur la pupille arrière de l'objectif (6)

3.   Dispositif selon la revendication 1 ou 2, **caractérisé en ce que** l'ensemble scanner de lames comprend :

- une source de lumière blanche (11),
- un condenseur (12) pour guider la lumière blanche vers la lame (8),

et une caméra (13) pour produire une image à partir de la lumière provenant de la lame (8).

**4.** Dispositif selon l'une quelconque des revendications précédentes, **caractérisé en ce que** les moyens de détection (9, 10) comprennent un condenseur (12) et un détecteur (10) disposé en aval de la lame (8) par rapport à la propagation du faisceau laser pulsé (3) de façon à détecter des signaux de transmission.

**5.** Dispositif selon l'une quelconque des revendications précédentes, **caractérisé en ce que** les moyens de détection (9, 10) comprennent un détecteur (9) disposé de façon à détecter des signaux réfléchis depuis la lame (8).

**6.** Dispositif selon l'une quelconque des revendications 3 à 5, **caractérisé en ce que** l'objectif et le condenseur (6, 12) sont respectivement disposés de part et d'autre de la lame (8).

**7.** Dispositif selon l'une quelconque des revendications précédentes, **caractérisé en ce que** l'objectif (6) présente une ouverture numérique supérieure à 0.5.

**8.** Dispositif selon l'une quelconque des revendications précédentes, **caractérisé en ce que** le faisceau laser puisé (3) est un train d'impulsions femtoseconde.

**9.** Dispositif selon l'une quelconque des revendications précédentes, caractérisé en ce le faisceau laser puisé présente un taux de répétition supérieur à une dizaine de MHz avec une puissance de plusieurs centaines de milliwatts.

**10.** Dispositif selon l'une quelconque des revendications précédentes, **caractérisé en ce que** le modulateur spatial de lumière (4) comprend un élément choisi parmi : un axicon; un élément diffractif ; un modulateur à cristaux liquides, et une lentille liquide modulable.

**11.** Dispositif selon l'une quelconque des revendications précédentes, **caractérisé en ce qu'**il comprend en outre des moyens de balayage du faisceau laser.

**12.** Application du dispositif selon l'une quelconque des revendications précédentes, pour l'imagerie quantitative du collagène comprenant la détection du signal de génération de second harmonique intrinsèque du collagène fibrillaire.

**13.** Application du dispositif selon l'une quelconque des revendications 1 à 11, pour l'imagerie quantitative du contenu lipidique d'un tissu comprenant la détection d'un signal de type génération de troisième harmonique, diffusion Raman anti-Stokes cohérente, diffusion Raman stimulée et/ou les mélanges à quatre ondes

**14.** Application du dispositif selon l'une quelconque des revendications 1 à 11, pour l'imagerie quantitative cellulaire ou extra cellulaire comprenant la détection d'un signal de fluorescence excitée à deux ou trois photons.

**Patentansprüche**

**1.** Optische Vorrichtung, umfassend:

- eine Scanner-Anordnung für histologische Objektträger, umfassend einen angetriebenen Objekttisch zum Tragen eines Objektträgers (8), auf dem eine Probe (7) angeordnet ist, und ein nichtlineares optisches Mikroskop, umfassend:

- eine Quelle (2) eines gepulsten Laserstrahls (3),
- einen räumlichen Lichtmodulator (4) zum Modulieren des räumlichen Profils des gepulsten Laserstrahls,
- ein Objektiv (6) zum Leiten des modulierten Strahls (5) zu dem Objektträger (8), und
- Detektionsmittel (9, 10) zum Auffangen kohärenter und inkohärenter Signale, die von der Probe (7) kommen, wobei der räumliche Lichtmodulator (4) ausgelegt ist, die Intensität und/oder Phase des gepulsten Laserstrahls (3) auf der hinteren Pupille des Objektivs (6) zu modulieren, um nach der Fokussierung durch das Objektiv (6) einen Strahl zu erzeugen, der axial ausgedehnt und in eine oder zwei seitliche Richtungen beschränkt ist.

**2.** Vorrichtung nach Anspruch 1, **dadurch gekennzeichnet, dass** der modulierte Strahl ein Bessel-Strahl (5) ist, der in Form einer ringförmigen Intensitätsverteilung auf der hinteren Pupille des Objektivs (6) erzeugt wird.

**3.** Vorrichtung nach Anspruch 1 oder 2, **dadurch gekennzeichnet, dass** die Scanner-Anordnung für Objektträger Folgendes umfasst:

- eine Weißlichtquelle (11),
- einen Kondensor (12) zum Leiten des Weißlichts zu dem Objektträger (8),

und eine Kamera (13) zur Erzeugung eines Bilds anhand des Lichts, das von dem Objektträger (8) kommt.

4. Vorrichtung nach einem der vorhergehenden Ansprüche, **dadurch gekennzeichnet, dass** die Detektionsmittel (9, 10) einen Kondensor (12) und einen Detektor (10), der bezogen auf die Ausbreitung des gepulsten Laserstrahls (3) vor dem Objektträger (8) angeordnet ist, umfassen, um Übertragungssignale zu detektieren.

5. Vorrichtung nach einem der vorhergehenden Ansprüche, **dadurch gekennzeichnet, dass** die Detektionsmittel (9, 10) einen Detektor (9) umfassen, der derart angeordnet ist, dass Signale detektiert werden, die von dem Objektträger (8) reflektiert werden.

6. Vorrichtung nach einem der Ansprüche 3 bis 5, **dadurch gekennzeichnet, dass** das Objektiv und der Kondensor (6, 12) jeweils beiderseits von dem Objektträger (8) angeordnet sind.

7. Vorrichtung nach einem der vorhergehenden Ansprüche, **dadurch gekennzeichnet, dass** das Objektiv (6) eine digitale Öffnung größer als 0.5 aufweist.

8. Vorrichtung nach einem der vorhergehenden Ansprüche, **dadurch gekennzeichnet, dass** der gepulste Laserstrahl (3) eine Folge von Femtosekundenimpulsen ist.

9. Vorrichtung nach einem der vorhergehenden Ansprüche, **dadurch gekennzeichnet, dass** der gepulste Laserstrahl eine Wiederholungsrate größer als etwa zehn MHz mit einer Leistung von mehreren hundert Milliwatt aufweist.

10. Vorrichtung nach einem der vorhergehenden Ansprüche, **dadurch gekennzeichnet, dass** der räumliche Lichtmodulator (4) ein Element umfasst, das aus Folgendem ausgewählt ist: einem Axicon; einem diffraktiven Element; einem Flüssigkristallmodulator und einer modulierbaren Flüssiglinse.

11. Vorrichtung nach einem der vorhergehenden Ansprüche, **dadurch gekennzeichnet, dass** sie ferner Mittel zur Abtastung des Laserstrahls umfasst.

12. Anwendung der Vorrichtung nach einem der vorhergehenden Ansprüche für die quantitative Abbildung des Kollagens, umfassend die Detektion des Signals einer Erzeugung der zweiten Harmonischen, das dem fibrillären Kollagen inhärent ist.

13. Anwendung der Vorrichtung nach einem der Ansprüche 1 bis 11 für die quantitative Abbildung des lipidischen Inhalts eines Gewebes, umfassend die Detektion eines Signals des Typs der Erzeugung der dritten Harmonischen, der kohärenten Anti-Stokes-Raman-Streuung, der stimulierten Raman-Streuung und/oder Vier-Wellen-Mischungen.

14. Anwendung der Vorrichtung nach einem der Ansprüche 1 bis 11 für die zelluläre oder extrazelluläre quantitative Abbildung, umfassend die Detektion eines Signals einer Zwei- oder Dreiphotonen-Fluoreszenzanregung.

**Claims**

1. Optical device comprising:

- a scanner assembly for histological slides comprising a motorized platform, which platform is intended to bear a slide (8) on which a sample (7) is placed, and a nonlinear optical microscope comprising:

- a pulsed laser beam (3) source (2);
- a spatial light modulator (4) for modulating the spatial profile of the pulsed laser beam;
- an objective (6) for guiding the modulated beam (5) to the slide (8); and
- detection means (9, 10) for collecting coherent and incoherent signals arising from the sample (7),

in which the spatial light modulator (4) is designed to modulate the intensity and/or the phase of the pulsed laser beam (3) on the back pupil of the objective (6) so as to produce a beam that is extended axially and confined in one or two lateral directions after focusing by the objective (6).

2. Device according to Claim 1, **characterized in that** the modulated beam is a Bessel beam (5) produced in the form of an annular intensity distribution on the back pupil of the objective (6).

3. Device according to Claim 1 or 2, **characterized in that** the slide scanner assembly comprises:

- a source of white light (11);
- a condenser (12) for guiding the white light to the slide (8),

and a camera (13) for producing an image on the basis of the light arising from the slide (8).

4. Device according to any one of the preceding claims, **characterized in that** the detection means (9, 10) comprise a condenser (12) and a detector (10) that is positioned downstream of the slide (8) with respect to the propagation of the pulsed laser beam (3) so as to detect transmission signals.

5. Device according to any one of the preceding claims, **characterized in that** the detection means (9, 10) comprises a detector (9) that is positioned so as to detect signals that are reflected from the slide (8).

6. Device according to any one of Claims 3 to 5, **characterized in that** the objective and the condenser (6, 12) are positioned on either side of the slide (8), respectively.

7. Device according to any one of the preceding claims, **characterized in that** the objective (6) has a numerical aperture that is larger than 0.5.

8. Device according to any one of the preceding claims, **characterized in that** the pulsed laser beam (3) is a femtosecond pulse train.

9. Device according to any one of the preceding claims, **characterized in that** the pulsed laser beam has a repetition rate that is higher than approximately 10 MHz with a power of several hundred milliwatts.

10. Device according to any one of the preceding claims, **characterized in that** the spatial light modulator (4) comprises an element chosen from: an axicon; a diffractive element; a liquid-crystal modulator, and a liquid lens that can be modulated.

11. Device according to any one of the preceding claims, **characterized in that** it further comprises means for scanning the laser beam.

12. Application of the device according to any one of the preceding claims, for the quantitative imaging of collagen comprising the detection of the second harmonic generation signal that is intrinsic to fibrillar collagen.

13. Application of the device according to any one of Claims 1 to 11, for the quantitative imaging of the lipid content of a tissue comprising the detection of a third harmonic generation, a coherent anti-Stokes Raman scattering, a stimulated Raman scattering and/or a four-wave mixing signal.

14. Application of the device according to any one of Claims 1 to 11, for cellular or extracellular quantitative imaging comprising the detection of a two- or three-photon excited fluorescence signal.

FIGURE 1

FIGURE 2

FIGURE 3

FIGURE 4

## RÉFÉRENCES CITÉES DANS LA DESCRIPTION

**Littérature non-brevet citée dans la description**

- **PASCAL DUFOUR et al.** Two-photon excitation fluorescence microscopy with a high depth of field using an axicon. *Applied Optics,* 20 Décembre 2006, vol. 45 (36
- **E. J. BOTCHERBY et al.** Scanning two photon fluorescence microscopy with extended depth of field. *Optics Communications,* 2006, vol. 268, 253-260

- **THOMAS A PLANCHON et al.** Rapid three-dimensional isotropic imaging of living cells using Bessel beam plane illumination. *Nature Methods,* Mai 2011, vol. 8 (5
- **NICOLAS OLIVIER et al.** Two-photon microscopy with simultaneous standard and extended depth of field using a tunable acoustic gradient-index lens. *Optics Letters,* 01 Juin 2009, vol. 34 (11
- **L. NOVOTNY ; B. HECHT.** Principles of nano-optics. Cambridge Univ Press, 2006